# EUROPEAN PATENT APPLICATION

(11) **EP 3 760 206 A1**
(43) Date of publication of application: **06.01.2021**
(21) Application number: 19760568.6
(22) Date of filing: 27.02.2019
(51) Int. Cl.: A61K 31/496, A61K 9/10, A61K 47/10, A61K 47/18, A61K 47/26, A61K 47/32, A61K 47/36, A61K 47/38, A61K 47/42, A61P 25/00, A61P 25/18, A61P 25/24, A61P 25/28

(54) **AQUEOUS SUSPENSION-TYPE PHARMACEUTICAL PREPARATION HAVING CONTROLLED DISSOLUTION**

(30) Priority: 28.02.2018 JP 2018035463
(71) Applicant: Sumitomo Dainippon Pharma Co., Ltd., Chuo-ku Osaka-shi Osaka 541-8524 (JP)
(72) Inventor: TSUZUKU, Takuma, Tokyo 104-8356 (JP)
(74) Representative: Vossius & Partner Patentanwälte Rechtsanwälte mbB
(86) International application number: PCT/JP2019/007424
(87) International publication number: WO 2019/167978

(57) **Abstract**

The present invention provides an aqueous suspension pharmaceutical preparation comprising the following (1) to (4) :
(1) (3aR,4S,7R,7aS)-2-{(1R,2R)-2-[4-(1,2-benzisothiazol-3-yl)piperazin-1-ylmethyl]cyclohexylmethyl}hexahydro-4,7-methano-2H-isoindole-1,3-dione (Compound 1), a pharmaceutically acceptable acid addition salt thereof, or a mixture thereof,
(2) xanthan gum,
(3) a dispersant, and
(4) water,
which can inhibit rapid dissolution and absorption of the drug even when the pharmaceutical preparation is administered orally and reduce the risk of unexpected side effects caused by the temporary increase in blood level of the drug.

## Description

### TECHNICAL FIELD

The present invention relates to an aqueous suspension pharmaceutical preparation. In detail, the present invention relates to an aqueous suspension pharmaceutical preparation comprising (3aR,4S,7R,7aS)-2-{(1R,2R)-2-[4-(1,2-benzisothiazol-3-yl)piperazin-1-ylmethyl]cyclohexylmethyl}hexahydro-4,7-methano-2H-isoindol-1,3-dione (hereinafter also referred to as "Compound 1"), a pharmaceutically acceptable acid addition salt thereof, or a mixture thereof (hereinafter also referred to as "Compound 1 or salt thereof") which is suspended in a solvent including water. Preferably, the present invention relates to the preparation for using as a medicine.

### BACKGROUND ART

It is well known that (3aR,4S,7R,7aS)-2-{(1R,2R)-2-[4-(1,2-benzisothiazol-3-yl)piperazin-1-ylmethyl]cyclohexylmethyl}hexahydro-4,7-methano-2H-isoindol-1,3-dione (Compound 1) is a compound used as an atypical antipsychotic agent, and the compound is useful in the treatment of a psychiatric disease such as schizophrenia, bipolar disorder, and depression (e.g. Patent Documents 1 to 3) .

In general, an oral solid preparation such as tablet and capsule has been widely used in the treatment of various diseases because it is easy for patients to take medicine at home. On the other hand, there are some situations which cannot be sufficiently treated by only the use of an oral solid preparation since the psychiatric disease such as schizophrenia causes various symptoms for each patient, in case of, for example, the administration to a patient suffering from acute schizophrenia, the administration to a patient in particular need of adjusting a dosage like children and senior people, the administration to a patient who refuses to take a drug, and the administration to a patient who has difficulty in swallowing. Recently, the number of senior people is increasing, and thus it has been strongly desired in medical field to provide an oral solution and an injection as a preparation with the improved medication compliance that reduces the burden for the senior patient's group.

In general, it has been known that an orally-administered drug must be in the solution state before the drug is absorbed, that is, before the drug passes through the gastrointestinal tract membrane. Hence, an oral solid preparation such as tablet is absorbed via the disintegration, dispersion and dissolution processes after administration. On the other hand, an oral solution is different from the oral solid preparation in the process until a drug is absorbed. A solution preparation, in which a drug is in the solution state, is absorbed without going through the disintegration, dispersion and dissolution processes until the drug is absorbed of oral solid preparation. Also, a suspension preparation, in which a drug is in the dispersed state, is absorbed without going through the disintegration process of oral solid preparation (Non-Patent Document 1). Hence, when an oral solution is designed, it is often necessary to study the design of the oral solution in view of the pharmacokinetic points different from oral solid preparations.

As an oral solution of Compound 1, a solution preparation comprising N-[4-[4-(1,2-benzisothiazol-3-yl)-1-piperazinyl]-(2R,3R)-2,3-tetramethylene-butyl]-(1'R,2'S,3'R,4'S)-2,3-bicyclo[2,2,1]heptanedicarboxyimide hydrochloride which is Compound 1 hydrochloride as an active ingredient and at least one substance selected from benzyl alcohol, N,N-dimethylacetamide, lactic acid or propylene glycol has ever been reported (Patent Document 4). However, Patent Document 4 focuses on solubilizing the compound at high concentrations. Patent Document 4 discloses that a solution preparation has characteristic solubility and stability, but does not disclose that the features are studied in view of the pharmacokinetic points. In addition, Patent Document 4 neither discloses nor suggests any suspension preparation.

Patent Document 5 discloses a composition comprising N-[4-[4-(1,2-benzisothiazol-3-yl)-1-piperazinyl]-(2R,3R)-2,3-tetramethylene-butyl]-(1'R,2'S,3'R,4'S)-2,3-bicyclo[2,2,1]heptanedicarboxyimide which is Compound 1, or a pharmaceutically acceptable acid addition salt thereof in the form of suspended particle, and more specifically a sustained-release preparation for injection which maintains an effective blood level of the compound. However, Patent Document 5 does not disclose that the preparation is administered orally. That is, Patent Document 5 neither discloses nor suggests any feature such as solubility in the stomach and absorbability when the preparation is administered orally.

### PRIOR ART DOCUMENTS

### PATENT DOCUMENTS

Patent Document 1: JP 2800953 B (US 5532372 A)
Patent Document 2: WO 2005/009999
Patent Document 3: WO 2006/126681
Patent Document 4: WO 2006/134864
Patent Document 5: WO 2012/053654

### NON-PATENT DOCUMENTS

Non-Patent Document 1: Biopharmaceuticals that can be understood in practice (First Edition), Hirokawa Shoten, 2009, p31-32

### SUMMARY OF INVENTION

### (PROBLEM TO BE SOLVED BY THE INVENTION)

It is assumed that an oral solution such as solution preparation and suspension preparation has fewer processes until a drug is absorbed as compared to an oral solid preparation, and thus the time until the drug is absorbed is shortened. In fact, it has been demonstrated that the tablet comprising Compound 1 or salt thereof prepared according to the process described in Patent Document 3 is not rapidly disintegrated in the dissolution test solution 1 described in the Japanese Pharmacopoeia 17th Edition and in the solution prepared by diluting the dissolution test solution with water. Hence, it has been thought that the oral solution, which does not go through the disintegration process, is absorbed in a shorter time after oral administration as compared to tablet and the blood level is rapidly increased, and the rapid increase in blood level of the drug may cause any disadvantages such as side effects.

In the stomach during fasting, a preparation passes through the stomach in a short time, and thus the disintegration process of the preparation in the stomach is highly likely to directly affect the absorbability of a drug. It is predicted that an oral solution comprising Compound 1 or salt thereof is often administered to a patient suffering from schizophrenia in the acute phase, and it is highly likely that such administration will be performed with or without eating. Hence, in order to reduce the risk of side effects, it is the problem to be solved to inhibit the absorbability of the oral solution in the stomach during fasting and suppress the significant increase in blood level of the drug.

The present invention has been studied considering the above situation, and an object of the present invention is to provide a suspension preparation comprising Compound 1 or salt thereof as an active ingredient wherein the suspension preparation does not produce more rapid dissolution and absorption as compared to oral solid preparation even when the preparation is administered orally.

### (MEANS FOR SOLVING THE PROBLEMS)

The present inventors have extensively studied to reach the above object, and then have found that the addition of (1) xanthan gum or (2) xanthan gum and an acidic polymer (or salt thereof), a sugar alcohol, or a mixture thereof into the suspension preparation comprising Compound 1 or salt thereof inhibits the dispersion of the suspension preparation in the test solution simulating the fasted stomach. In general, when a suspension preparation is administered in the test solution in the dissolution test, the suspended particle in the preparation is rapidly dispersed. On the other hand, it was observed in the preferable suspension preparation of the present invention that the clump of Compound 1 or salt thereof was formed in the test solution, the clump was gradually disintegrated from the surface thereof such that a tables is gradually disintegrated, and then the dispersion of the suspended particle in the test solution was inhibited. The present inventors have found that the inhibition of the dispersion of the suspension preparation is achieved via the pseudo-disintegration process in the stomach like the disintegration process of oral solid preparations, and thus leads to the initial dissolution inhibition of the preparation. Based upon the new findings, the present invention has been completed.

The present invention provides inventions described below.

[Item 1] An aqueous suspension pharmaceutical preparation comprising the following (1) to (4):
(1) (3aR,4S,7R,7aS)-2-{(1R,2R)-2-[4-(1,2-benzisothiazol-3-yl)piperazin-1-ylmethyl]cyclohexylmethyl}hexahydro-4,7-methano-2H-isoindole-1,3-dione (Compound 1), a pharmaceutically acceptable acid addition salt thereof, or a mixture thereof,
(2) xanthan gum,
(3) a dispersant, and
(4) water.

[Item 2] The pharmaceutical preparation according to the item 1, which comprises an acidic polymer or salt thereof, a sugar alcohol, or a mixture thereof besides the (1) to (4) defined in the item 1.

[Item 3] The pharmaceutical preparation according to the item 1 or 2, which comprises at least one ingredient selected from the group consisting of a cellulose derivative, a sucrose fatty acid ester, and polyvinyl alcohol as the dispersant.

[Item 4] The pharmaceutical preparation according to the item 3, which comprises a cellulose derivative as the dispersant.

[Item 5] The pharmaceutical preparation according to the item 1 or 2, which comprises at least one ingredient selected from the group consisting of hypromellose, hydroxypropylcellulose, and hydroxyethyl cellulose as the dispersant.

[Item 6] The pharmaceutical preparation according to the item 5, which comprises hypromellose as the dispersant.

[Item 7] The pharmaceutical preparation according to any one of the items 2 to 6, which comprises at least one ingredient selected from the group consisting of (i) at least one acidic polymer or salt thereof selected from the group consisting of alginic acid, carrageenan, gelatin, guar gum, and pectin, and (ii) at least one sugar alcohol selected from the group consisting of erythritol and sorbitol.

[Item 8] The pharmaceutical preparation according to the item 7, which comprises at least one ingredient selected from the group consisting of sodium alginate and erythritol.

[Item 9] The pharmaceutical preparation according to any one of the items 1 to 8 with a pH of 2.5 - 5.5.

[Item 10] The pharmaceutical preparation according to any one of the items 1 to 9, which further comprises (5) one or more chlorides selected from an inorganic chloride or a quaternary ammonium chloride having 4 to 12 carbon atoms.

[Item 11] The pharmaceutical preparation according to any one of the items 1 to 10, wherein the concentration of xanthan gum is about 3 mg/mL - about 15 mg/mL.

[Item 12] The pharmaceutical preparation according to any one of the items 1 to 10, wherein the concentration of xanthan gum is about 4 mg/mL to 9 mg/mL.

[Item 13] The pharmaceutical preparation according to any one of the items 1 to 12, wherein the content of Compound 1, a pharmaceutically acceptable acid addition salt thereof, or a mixture thereof is about 5 - about 120 mg/mL in terms of Compound 1 hydrochloride.

[Item 14] The pharmaceutical preparation according to any one of the items 1 to 12, wherein the content of Compound 1, a pharmaceutically acceptable acid addition salt thereof, or a mixture thereof is about 10 - about 100 mg/mL in terms of Compound 1 hydrochloride.

[Item 15] The pharmaceutical preparation according to any one of the items 1 to 12, wherein the content of Compound 1, a pharmaceutically acceptable acid addition salt thereof, or a mixture thereof is about 20 - about 80 mg/mL in terms of Compound 1 hydrochloride.

[Item 16] The pharmaceutical preparation according to any one of the items 1 to 15, wherein Compound 1, a pharmaceutically acceptable acid addition salt thereof, or a mixture thereof is Compound 1 hydrochloride.

[Item 17] The pharmaceutical preparation according to any one of the items 1 to 16 for oral administration.

[Item 18] The pharmaceutical preparation according to any one of the items 1 to 17, wherein the dissolution ratio of Compound 1, a pharmaceutically acceptable acid addition salt thereof, or a mixture thereof at 5 minutes after the start of the test performed under the following test condition according to the dissolution test (the paddle method) described in the Japanese Pharmacopoeia 17th Edition is 70% or less.

### [Test Condition]

Test solution: Mixture of the dissolution test solution 1 described in the Japanese Pharmacopoeia 17th Edition (50 mL) and water (200 mL)
Volume of test solution: 250 mL
Temperature of test solution: 36.5 - 37.5°C
Rotation speed: 50 rpm
Test preparation: Preparation comprising 40 mg of Compound 1, a pharmaceutically acceptable acid addition salt thereof, or a mixture thereof in terms of Compound 1 hydrochloride

[Item 19] The pharmaceutical preparation according to any one of the items 1 to 17, wherein the dissolution ratio of Compound 1, a pharmaceutically acceptable acid addition salt thereof, or a mixture thereof at 5 minutes after the start of the dissolution test according to the item 18 is 60% or less.

[Item 20] The pharmaceutical preparation according to any one of the items 1 to 17, wherein the dissolution ratio of Compound 1, a pharmaceutically acceptable acid addition salt thereof, or a mixture thereof at 5 minutes after the start of the dissolution test according to the item 18 is 50% or less .

[Item 21] A medicament for treatment and/or prophylaxis of a psychiatric disease, which comprises the pharmaceutical preparation according to any one of the items 1 to 20.

[Item 22] The medicament according to the item 21, wherein the psychiatric disease is schizophrenia, bipolar disorder, senile dementia, or depression.

[Item 23] A method of treating and/or preventing a psychiatric disease, which comprises administering the pharmaceutical preparation according to any one of the items 1 to 20 to a patient in need thereof.

[Item 24] The method according to the item 23, wherein the psychiatric disease is schizophrenia, bipolar disorder, senile dementia, or depression.

[Item 25] Use of the pharmaceutical preparation according to any one of the items 1 to 20 in the manufacture of a medicament for treatment and/or prophylaxis of a psychiatric disease.

[Item 26] The use according to the item 26, wherein the psychiatric disease is schizophrenia, bipolar disorder, senile dementia, or depression.

[Item 27] The pharmaceutical preparation according to any one of the items 1 to 20 for use in the treatment and/or prophylaxis of a psychiatric disease.

[Item 28] The pharmaceutical preparation according to the item 27, wherein the psychiatric disease is schizophrenia, bipolar disorder, senile dementia, or depression.

[Item 29] An agent for controlling dissolution of an aqueous suspension pharmaceutical preparation, comprising xanthan gum.

[Item 30] The agent according to the item 29, wherein the dissolution control is the inhibition of the initial dissolution rate.

[Item 31] The agent according to the item 29, wherein the dissolution control is the inhibition of the initial dissolution rate in the solution at a pH of 1.0 - 2.5.

[Item 32] The agent according to any one of the items 29 to 31, which further comprises one ingredient selected from an acidic polymer or salt thereof or a sugar alcohol, or a mixture thereof.

[Item 33] An agent for controlling dissolution of an aqueous suspension pharmaceutical preparation with a pH of 2.5 - 5.5 comprising the following (1) to (4):
(1) (3aR,4S,7R,7aS)-2-{(1R,2R)-2-[4-(1,2-benzisothiazol-3-yl)piperazin-1-ylmethyl]cyclohexylmethyl}hexahydro-4,7-methano-2H-isoindole-1,3-dione (Compound 1), a pharmaceutically acceptable acid addition salt thereof, or a mixture thereof,
(2) one or more chlorides selected from an inorganic chloride or a quaternary ammonium chloride having 4 to 12 carbon atoms,
(3) a dispersant, and
(4) water,
wherein the agent for controlling dissolution comprises xanthan gum.

[Item 34] The agent according to the item 33, which further comprises an acidic polymer or salt thereof, a sugar alcohol, or a mixture thereof.

[Item 35] A method of controlling the dissolution of an aqueous suspension pharmaceutical preparation, which comprises adding xanthan gum to the aqueous suspension pharmaceutical preparation.

[Item 36] The method according to the item 35, wherein the dissolution control is the inhibition of the initial dissolution rate.

### (EFFECTS OF THE INVENTION)

The present invention shows the similar absorption process to tablet and capsule in the simulated stomach when the aqueous suspension pharmaceutical preparation comprising Compound 1 or salt thereof as an active ingredient is administered orally, and thus can inhibit rapid dissolution and absorption of the pharmaceutical preparation. Also, the present invention can reduce the risk of unexpected side effects caused by the temporary increase in blood level of the drug.

### DESCRIPTION OF EMBODIMENTS

Hereinafter, the preferred embodiments of the present invention are explained in detail. However, the present invention is not limited to the following embodiments.

The present invention discloses an aqueous suspension pharmaceutical preparation of Compound 1 or salt thereof suitable for oral administration as a medicament for the treatment of a disease such as schizophrenia.

The term "aqueous suspension pharmaceutical preparation" comprising Compound 1 or salt thereof as an active ingredient as used herein means a preparation in which the suspended particle of Compound 1 or salt thereof is dispersed in a solvent including water as main solvent. The "aqueous suspension pharmaceutical preparation" as used herein may comprise a polar solvent in an amount which produces no negative effect (e.g. bitter taste, astringent taste, astringent) on administration feeling of the preparation besides water.

The term "polar solvent" as used herein means a solvent with polarity which can be homogeneously mixed with water.

(3aR,4S,7R,7aS)-2-{(1R,2R)-2-[4-(1,2-benzisothiazol-3-yl)piperazin-1-ylmethyl]cyclohexylmethyl}hexahydro-4,7-methano-2H-isoindole-1,3-dione (Compound 1) of the present invention is a compound of the following formula:

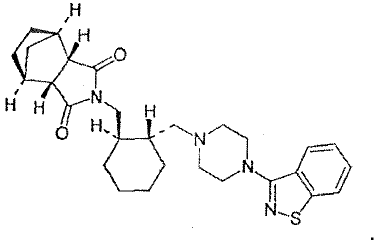

Compound 1 or salt thereof has the pharmacological effect of an antipsychotic agent. More specifically, Compound 1 or salt thereof and a pharmaceutical preparation comprising the same are useful as a medicament for the treatment of a psychiatric disease such as schizophrenia, bipolar disorder, and depression.

Examples of the pharmaceutically acceptable acid addition salt of Compound 1 include an organic acid addition salt and an inorganic acid addition salt. Examples of the organic acid addition salt include acetate, lactate, adipate, citrate, tartrate, methanesulfonate, fumarate, and maleate, and examples of the inorganic acid addition salt include hydrochloride, sulfate, nitrate, and phosphate, but are not limited thereto. The pharmaceutically acceptable acid addition salt of Compound 1 is preferably hydrochloride. In addition, Compound 1 and a pharmaceutically acceptable acid addition salt thereof may be in the form of a solvate, a hydrate or a nonhydrate.

Compound 1 or pharmaceutically acceptable acid addition salt thereof can be prepared according to, for example, the processes of Patent Documents 1 and 2 or similar processes thereto. The prepared Compound 1 or pharmaceutically acceptable acid addition salt thereof may be milled according to a commonly-used method as appropriate.

The term "mixture thereof" in "(3aR,4S,7R,7aS)-2-{(1R,2R)-2-[4-(1,2-benzisothiazol-3-yl)piperazin-1-ylmethyl]cyclohexylmethyl}hexahydro-4,7-methano-2H-isoindole-1,3-dione, a pharmaceutically acceptable acid addition salt thereof, or a mixture thereof" as used herein encompasses (1) a mixture of Compound 1 (in the free form) and a pharmaceutically acceptable acid addition salt of Compound 1 (which may be one or two or more acid addition salts), (2) a mixture of two or more pharmaceutically acceptable acid addition salts of Compound 1.

The content of "(3aR,4S,7R,7aS)-2-{(1R,2R)-2-[4-(1,2-benzisothiazol-3-yl)piperazin-1-ylmethyl]cyclohexylmethyl}hexahydro-4,7-methano-2H-isoindole-1,3-dione, a pharmaceutically acceptable acid addition salt thereof, or a mixture thereof" as used herein varies according to the amount of drug solution, but the content thereof in the preparation is basically about 0.1 - about 400 mg/mL in terms of Compound 1 hydrochloride. The content of "Compound 1 or salt thereof" is preferably about 1 - about 300 mg/mL, more preferably about 5 - about 120 mg/mL, furthermore preferably about 10 - about 100 mg/mL, even more preferably about 20 - about 80 mg/mL, particularly preferably about 25 - about 80 mg_{/}mL, and most preferably about 25 - about 50 mg_{/}mL, in terms of Compound 1 hydrochloride. The term "in terms of Compound 1 hydrochloride" as used herein means that the amount of Compound 1, a pharmaceutically acceptable acid addition salt of Compound 1 or a hydrate or solvate thereof is calculated by substituting the amount thereof with the amount of Compound 1 hydrochloride in an equimolar amount thereto.

In the present invention, xanthan gum is used as an agent for controlling dissolution which produces the effect of controlling rapid dissolution and absorption of an aqueous suspension preparation in the stomach during fasting, and can produce the pseudo-disintegration process of the aqueous suspension preparation in the stomach. The agent for controlling dissolution of the present invention is not particularly limited as long as it is used as an additive for a conventional suspension preparation.

Examples of the agent for controlling dissolution include an acidic polymer or salt thereof and a sugar alcohol. Examples of the acidic polymer or salt thereof include sodium alginate, carrageenan, gelatin, guar gum and pectin, and examples of the sugar alcohol include erythritol and sorbitol. The agent for controlling dissolution may also be used alone or in mixture of two or more agents for controlling dissolution.

The content of the agent for controlling dissolution is preferably about 0.5 - about 20 mg/mL, more preferably about 1 - about 15 mg/mL, and furthermore preferably about 2 - about 10 mg/mL of the total amount of the preparation, but is not limited thereto.

The term "chloride" as used herein means an inorganic chloride or a quaternary ammonium chloride having 4 to 12 carbon atoms.

Examples of the inorganic chloride include sodium chloride, potassium chloride, iron (II) chloride, calcium chloride, magnesium chloride, zinc chloride, and ammonium chloride. The chloride is preferably sodium chloride and potassium chloride, and more preferably sodium chloride.

The quaternary ammonium chloride having 4 to 12 carbon atoms is not particularly limited as long as it is an organic quaternary ammonium chloride in a range of 4 to 12 carbon atoms in total. The quaternary ammonium chloride is preferably an organic quaternary ammonium chloride with a group such as straight or branched alkyl and alkylene (wherein the quaternary ammonium moiety may have a group such as hydroxy, amino and acyl), and particularly preferably choline chloride.

The concentration of chloride ion in the preparation is about 0.015 - about 1 mol/L, preferably about 0.05 - about 1 mol/L, more preferably about 0.10 - about 1 mol/L, but is not limited thereto. The concentration of chloride ion in the preparation is free of the chloride ion derived from a salt of Compound 1.

The "dispersant" as used herein is not particularly limited as long as it is used as an additive for a conventional suspension preparation. Examples thereof include a cellulose derivative such as cellulose, hypromellose (hydroxypropylmethylcellulose), hydroxypropylcellulose, hydroxyethyl cellulose, methylcellulose, ethylcellulose, and carmellose sodium; an alkyl alcohol such as polyvinyl alcohol; a sucrose fatty acid ester such as sucrose myristate, sucrose laurate, and sucrose stearate; a polyglyceryl fatty acid ester such as polyglyceryl myristate, polyglyceryl laurate, and polyglyceryl stearate; a pyrrolidone derivative such as polyvinylpyrrolidone; a non-ionic surfactant such as Poloxamer 188, HCO-60, Polysorbate 80, and Polyoxyl 40 Stearate; an ionic surfactant such as sodium lauryl sulfate. The dispersant is preferably a cellulose derivative, a sucrose fatty acid ester and an alkyl alcohol, more preferably a cellulose derivative, a sucrose fatty acid ester and polyvinyl alcohol, and furthermore preferably a cellulose derivative. Specific examples thereof preferably include hypromellose, hydroxypropylcellulose and hydroxyethyl cellulose, and more preferably hypromellose. The dispersant may also be used alone or in mixture of two or more dispersants.

The content of the dispersant is preferably about 0.01 - about 40 mg/mL of the total amount of the preparation, but is not limited thereto. More specifically, when the dispersant is a cellulose derivative such as hypromellose, hydroxypropylcellulose and hydroxyethyl cellulose or a sucrose fatty acid ester such as sucrose laurate, the content thereof is preferably about 0.1 - about 30 mg/mL, more preferably about 0.1 - about 20 mg/mL, and furthermore preferably about 0.1 - about. 10 mg/mL of the total amount of the preparation, but is not limited thereto. Also, when the dispersant is an alkyl alcohol such as polyvinyl alcohol, the content thereof is preferably about 0.1 - about 10 mg/mL, and more preferably about 0.5 - about 5 mg/mL of the total amount of the preparation, but is not limited thereto. When the dispersant is a polyglyceryl fatty acid ester, a pyrrolidone derivative, a non-ionic surfactant or an ionic surfactant, the content thereof is preferably about 0.01 - about 0.5 mg/mL, and more preferably about 0.01 - about 0.1 mg/mL of the total amount of the preparation, but is not limited thereto.

The pH of the aqueous suspension pharmaceutical preparation of the present invention is 2.5 - 5.5, preferably 3.0 - 5.5, and more preferably 3.0 - 5.0.

In the aqueous suspension pharmaceutical preparation of the present invention, the dissolution ratio of Compound 1 or salt thereof in the test solution simulating the fasted stomach can be measured. The dissolution ratio of Compound 1 or salt thereof in the test solution simulating the fasted stomach in the aqueous suspension pharmaceutical preparation of the present invention can be measured by, for example, the following dissolution test A in which the dissolution test (the paddle method) described in the Japanese Pharmacopoeia 17th Edition is partially changed.

### Dissolution Test A:

A mixture of the dissolution test solution 1 described in the Japanese Pharmacopoeia 17th Edition (50 mL) and water (200 mL) (hereinafter referred to as "test solution") is kept at 36.5 - 37.5°C, the preparation comprising 40 mg of Compound 1 or salt thereof in terms of Compound 1 hydrochloride is added into the test solution, the paddle is rotated in the test solution at 50 rpm, the test solution is sampled at 5 minutes, 10 minutes, 15 minutes and 30 minutes after the start of the test, and the concentration of Compound 1 or salt thereof dissolved in the test solution is measured. The concentration thereof measured for the test solution is expressed as a percentage value with respect to the dissolution concentration when completely dissolved (40 mg/250 mL), and the value is defined as dissolution ratio. The water used for the test solution is not particularly limited. The water may be selected from purified water, tap water or mineral water unless otherwise specified. The water is preferably tap water or mineral water used for usual administration.

When the dissolution ratio of the aqueous suspension pharmaceutical preparation of the present invention is measured according to the above dissolution test A, the dissolution ratio of Compound 1 or salt thereof at 5 minutes after the start of the test is, for example, 70% or less, preferably 60% or less, and more preferably 50% or less in terms of Compound 1 hydrochloride. Also, the dissolution ratio of Compound 1 or salt thereof at 10 minutes after the start of the test is, for example, 95% or less, preferably 80% or less, and more preferably 70% or less in terms of Compound 1 hydrochloride.

The term "inhibit(s) the initial dissolution rate" by the addition of the "agent for controlling dissolution" of the present invention as used herein means that the initial dissolution ratio is reduced as compared to the dissolution ratio of the preparation prior to the addition of the agent for controlling dissolution.

For example, it means that when the dissolution rate of the aqueous suspension pharmaceutical preparation is measured according to the above dissolution test A, the dissolution ratio of Compound 1 or salt thereof in the pharmaceutical preparation at 5 minutes after the start of the test is smaller as compared to that of the preparation comprising no "agent for controlling dissolution" of the present invention. Preferably, the term "inhibit(s) the initial dissolution rate" means that the dissolution ratio of Compound 1 or salt thereof at 5 minutes after the start of the test is, for example, 70% or less, preferably 60% or less, and more preferably 50% or less in terms of Compound 1 hydrochloride.

When the aqueous suspension pharmaceutical preparation comprising Compound 1 or salt thereof as an active ingredient is administered orally, the present invention can inhibit rapid dissolution and absorption of the drug. Also, the present invention can reduce the risk of unexpected side effects caused by the temporary increase in blood level of the drug. In addition, in the preferable embodiment of the present invention, the aqueous suspension pharmaceutical preparation can easily assure the bioequivalence with the existing tablets (e.g. the tablet described in Patent Document 3, the tablet of Compound 1 hydrochloride sold in the US since 2011).

The term "D50" as used herein is one of the representative indexes for representing the particle size distribution of the suspended particle of Compound 1 or salt thereof in the aqueous suspension pharmaceutical preparation and means 50% particle size (median diameter) . The D50 of the suspended particle in the aqueous suspension pharmaceutical preparation of the present invention is about 0.1 - about 13 µm, preferably about 0.1 - about 10 µm, and more preferably about 0.1 - about 7 µm, but is not limited thereto.

The term "D90" as used herein means 90% particle size. The D90 of the suspended particle in the aqueous suspension pharmaceutical preparation of the present invention is about 1 - about 30 µm, preferably about 1 - about 20 µm, and more preferably about 1 - about 15 µm, but is not limited thereto.

The suspended particle of Compound 1 or salt thereof whose D50 is about 0.1 - about 13 µm or D90 is about 1 - about 30 µm can be produced by, for example, dry milling Compound 1 or salt thereof using a mill such as jet mill and hammer mill, but the present invention is not limited thereto. Also, the suspended particle can be produced by adding Compound 1 or salt thereof into a solvent, and then wet-milling the resulting product using a machine such as homomixer, high-speed rotation type disperser, high-pressure homogenizer and bead mill. In addition, the suspended particle can be produced by a commonly-used recrystallization technique of Compound 1 or salt thereof besides the method of milling Compound 1 or salt thereof.

As Compound 1 or salt thereof for using at the time of the manufacture of the aqueous suspension pharmaceutical preparation of the present invention, Compound 1 or salt thereof whose D50 is about 0.1 - about 13 µm or D90 is about 1 - about 30 µm produced by an operation such as dry-milling, wet-milling and recrystallization technique can be used, but is not limited thereto. Even if Compound 1 or salt thereof does not have a D50 of about 0.1 - about 13 µm or a D90 of about 1 - about 30 µm at the time of the manufacture of the aqueous suspension pharmaceutical preparation of the present invention, the suspended particle of Compound 1 or salt thereof whose D50 is about 0.1 - about 13 µm or D90 is about 1 - about 30 µm can be produced by an operation such as wet-milling in the manufacture step.

In general, the particle size is measured with an analyzer such as laser diffraction particle size analyzer, dynamic light scattering particle size analyzer, and image processing particle size analyzer. The D50 and D90 values as used herein can be calculated from the particle size distribution measured with laser diffraction particle size analyzer: HELOS BR-MULTI (Sympatec).

The aqueous suspension pharmaceutical preparation of the present invention may comprise a suspended particle other than the suspended particle of "(3aR, 4S, 7R, 7aS) -2-{(1R,2R)-2-[4-(1,2-benzisothiazol-3-yl)piperazin-1-ylmethyl]cyclohexylmethyl}hexahydro-4,7-methano-2H-isoindole-1,3-dione, a pharmaceutically acceptable acid addition salt thereof, or a mixture thereof (Compound 1 or salt thereof)" as "suspended particle". Such aqueous suspension pharmaceutical preparation is preferably a preparation wherein the amount of the suspended particle other than that of Compound 1 or salt thereof is an amount which does not affect any feature such as the absorbability of such preparation (including a preparation in which the amount is zero), and more preferably a preparation with no suspended particle other than that of Compound 1 or salt thereof.

The aqueous suspension pharmaceutical preparation of the present invention may comprise an additive for a conventional suspension preparation unless the effect of the present invention is lost. Examples of such additive include preservative, stabilizing agent, buffering agent, sweetening agent, coloring agent, and flavoring agent, but are not limited thereto.

The preservative is not particularly limited as long as it is used as an additive for a conventional suspension preparation. Examples thereof include a benzoic acid derivative such as methyl paraoxybenzoate, ethyl paraoxybenzoate, propyl paraoxybenzoate, butyl paraoxybenzoate, paraoxybenzoic acid, benzoic acid, and sodium benzoate; a compound of a backbone comprising three or more carbon atoms with one to four carboxyl groups such as sorbic acid, potassium sorbate, sodium edetate and citric acid; and an alcohol such as glycerin, ethanol, 2-propanol and propylene glycol. The preservative is preferably a benzoic acid derivative. Specific examples thereof preferably include methyl paraoxybenzoate, ethyl paraoxybenzoate, propyl paraoxybenzoate, butyl paraoxybenzoate and sodium benzoate, more preferably methyl paraoxybenzoate, propyl paraoxybenzoate and sodium benzoate, furthermore preferably methyl paraoxybenzoate and sodium benzoate, and most preferably methyl paraoxybenzoate. In addition, the preservative may be used alone or in a mixture of two or more preservatives.

The content of the preservative is preferably about 0.1 - about 10 mg/mL, more preferably about 0.2 - about 5 mg/mL, furthermore preferably about 0.25 - about 3 mg/mL, and particularly preferably about 0.5 - about 2.5 mg/mL of the total amount of the preparation, but is not limited thereto.

The stabilizing agent is not particularly limited as long as it is used as an additive for a conventional suspension preparation. Examples thereof include an alcohol. Examples of the alcohol include a monohydric alcohol such as ethanol; a polyhydric alcohol such as glycerin, propylene glycol, and polyethylene glycol; a sugar alcohol such as sorbitol, erythritol, and mannitol. The stabilizing agent is preferably a polyhydric alcohol. Specific examples thereof preferably include glycerin, sorbitol, erythritol, and propylene glycol, and more preferably propylene glycol. In addition, the stabilizing agent can be used alone or in mixture of two or more stabilizing agents.

The content of the stabilizing agent is preferably about 1 - about 500 mg_{/}mL, more preferably about 10 - about 400 mg/mL, furthermore preferably about 30 - about 350 mg/mL, still furthermore preferably about 50 - about 300 mg_{/}mL, and particularly preferably about 50 - about 150 mg/mL of the total amount of the preparation, but is not limited thereto.

The buffering agent is not particularly limited as long as it is used as an additive for a conventional suspension preparation. Examples thereof include sodium salt, potassium salt, and hydrate thereof. Examples of the sodium salt include disodium phosphate, monosodium phosphate, sodium hydrogen carbonate, sodium carbonate, sodium citrate, and hydrate thereof. Examples of the potassium salt include dipotassium phosphate, monopotassium phosphate, potassium hydrogen carbonate, potassium carbonate, potassium citrate, and hydrate thereof. The buffering agent is preferably sodium salt and potassium salt. Specific examples thereof preferably include dipotassium phosphate, monopotassium phosphate, disodium phosphate, monosodium phosphate, and hydrate thereof, more preferably dipotassium phosphate, monopotassium phosphate, disodium phosphate, monosodium phosphate, and hydrate thereof, furthermore preferably dipotassium phosphate, monosodium phosphate, and hydrate thereof. In addition, the buffering agent may be used alone or in a mixture of two or more buffering agents.

Dipotassium phosphate is the same compound as dipotassium hydrogen phosphate, and monopotassium phosphate is the same compound as potassium dihydrogen phosphate.

The content of the buffering agent in the aqueous suspension pharmaceutical preparation of the present invention is preferably about 0.01 - about 15 mg, more preferably about 0.05 - about 5 mg, furthermore preferably about 0.125 - about 0.85 mg, and particularly preferably about 0.2 - about 0.5 mg relative to 1 mg of Compound 1 or salt thereof in the preparation (the weight in terms of Compound 1 hydrochloride), but is not limited thereto. When the content of the buffering agent is less than 0.01 mg relative to 1 mg of Compound 1 or salt thereof in the preparation (the weight in terms of Compound 1 hydrochloride), there is the possibility to produce the reduced stability and administration feeling of Compound 1 or salt thereof because of the increased dissolution amount of Compound 1 or salt thereof to a solvent. In the case that the aqueous suspension pharmaceutical preparation of the present invention comprises a chloride, another buffering agent may not be contained because the chloride acts as buffering agent. When the content of the buffering agent exceeds 15 mg relative to 1 mg of Compound 1 or salt thereof in the preparation (the weight in terms of Compound 1 hydrochloride), there is the possibility to make the maintenance of the suspended stare difficult because of the deposition of other additive such as a dispersant by salting out.

The sweetening agent is not particularly limited as long as it is used as an additive for a conventional suspension preparation. Examples thereof include sucralose, stevia, sucrose, liquid sugar, fructose, sorbitol, xylitol, erythritol, trehalose, maltitol, and acesulfame potassium. The sweetening agent is preferably sucralose, stevia, sorbitol, and erythritol, more preferably sucralose, stevia, and erythritol, and furthermore preferably sucralose and stevia. In addition, the sweetening agent may be used alone or in mixture of two or more sweetening agents.

The coloring agent is not particularly limited as long as it is used as an additive for a conventional suspension preparation. Examples thereof include tar dye, Yellow No.5, and caramel. In addition, the coloring agent may be used alone or in a mixture of two or more coloring agents.

The flavoring agent is not particularly limited as long as it is used as an additive for a conventional suspension preparation. Examples thereof include apple flavor, medical essence, lemon oil, orange oil, and peach oil. In addition, the flavoring agent may be used alone or in a mixture of two or more flavoring agents.

The aqueous suspension pharmaceutical preparation of the present invention can be prepared by the preparation process of a conventional oral solution. For example, the aqueous suspension pharmaceutical preparation of the present invention can be prepared by the process comprising the following steps (1)-(5), but is not limited thereto.
(1) A dispersant and other additive are added to a solvent and dissolved.
(2) To the solution prepared in the above (1) is added the dry-milled Compound 1 or salt thereof and mixed to prepare Solution A.
(3) As appropriate, the clump of Compound 1 or salt thereof added in the above (2) is loosened with a machine such as homomixer, stirring machine and high-speed rotation type disperser (wet-milling) or the clump is stirred or dispersed to homogeneously suspend Compound 1 or salt thereof.
(4) Separately, in another container, an additive other than a dispersant is added to a solvent and dissolved to prepare Solution B.
(5) The aqueous suspension pharmaceutical preparation of the present invention can be prepared by mixing Solution A and Solution B prepared in the above steps in an appropriate amount.

In the process comprising the above (1) - (5), it is preferable that Compound 1 or salt thereof and a dispersant are contained in Solution A, and an agent for controlling dissolution is contained in Solution B. On the other hand, other additives may be contained in Solution A, Solution B or both of Solution A and Solution B.

When an additive is added to a solvent and dissolved in the manufacture of the aqueous suspension pharmaceutical preparation of the present invention, the additive can also be dissolved with a heated solvent. Also, the additive is preliminarily dissolved in a solvent such as propylene glycol, ethanol and glycerin and the resulting solution may be mixed with a solvent. These processes are helpful when an additive with low solubility in a solvent is dissolved to the solvent. Examples of the additive with low solubility in a solvent include preservative, but are not limited thereto.

The aqueous suspension pharmaceutical preparation of the present invention have no specific disadvantage even if the preparation has air bubbles. The preparation process may be modified so than the preparation has no air bubble. Specifically, the aqueous suspension pharmaceutical preparation of the present invention in which no air bubble is visually contained can be prepared, for example, by appropriately modifying any preparation condition such as processing time, processing strength, liquid temperature of processed product and indoor pressure in the wet-milling, stirring or dispersion step, but the present invention is not limited thereto. Even if air bubbles are contained in the preparation in the preparation process, the contained air bubbles can be removed from the aqueous suspension pharmaceutical preparation of the present invention by the introduction of a step such as reduced pressure step and stirring step.

The storage container of the aqueous suspension pharmaceutical preparation of the present invention is not particularly limited as long as it is a storage container for a conventional oral solution or injection medicine. Examples thereof include a vial, an ample, a glass bottle, a polyethylene bottle and a container divided by aluminum multilayer film.

The aqueous suspension pharmaceutical preparation of the present invention can be prepared in an appropriate concentration depending on factors such as dosage, and administered by adjusting the dosage as appropriate.

Even if the aqueous suspension pharmaceutical preparation of the present invention is administered orally, the preparation has little effect on gastrointestinal tract motility and environment in the gastrointestinal tract. As a result, the aqueous suspension pharmaceutical preparation of the present invention can also be administered in emergency situations.

### EXAMPLES

Hereinafter, the present invention is explained in more detail in Examples, Comparative Examples, Test Examples, but should not be limited thereto.

In the following Examples and Comparative Examples, the following hypromellose, sodium chloride, propylene glycol, sodium benzoate, methylparaben, xanthan gum, dipotassium phosphate, sucralose, sodium alginate, erythritol, carrageenan, and gellan gum were used, but the present invention is not limited thereto.
- hypromellose: TC-5R (Shin-Etsu Chemical Co., Ltd.)
- sodium chloride: Sodium chloride suitable for the biopharmaceutical production (Merck Co., Ltd.)
- propylene glycol: Japanese Pharmacopoeia propylene glycol (ADEKA)
- sodium benzoate: Japanese Pharmacopoeia sodium benzoate (Fushimi Pharmaceutical Co., Ltd.)
- methylparaben: sodium paraoxybenzoate (Ueno Fine Chemicals Industry, Ltd.)
- xanthan gum: Echogum T/KELTROL T (DSP GOKYO FOOD & CHEMICAL Co., Ltd.)
- dipotassium phosphate: dipotassium hydrogen phosphate (Taihei Chemical Industrial Co., Ltd.)
- sucralose: Sucralose P (San-Ei Gen F.F.I, Inc.)
- sodium alginate: KIMICA ALGIN 1-5 (KIMICA Corporation)
- erythritol: Erythritol (Cargill)
- carrageenan: Carrageenin CSL-1 (San-Ei Gen F.F.I, Inc.)
- gellan gum: KELCOGEL (San-Ei Gen F.F.I, Inc.)

### Examples 1-3

According to the following procedure, the preparations of Examples 1-3 were prepared from Compound 1 hydrochloride, additives and a solvent in the amounts shown in Table below.

### (1) Preparation of Solution A

Hypromellose, sodium benzoate, sodium chloride and sucralose were added to purified water and dissolved. Stevia, erythritol, sodium alginate and apple flavor were then added thereto and dissolved. To the solution was then added Compound 1 hydrochloride, and the mixture was dispersed with Precision Dispersion/Emulsification machine: CLEARMIX CLM-0.8S (M Technique Co., Ltd.) at 8000 rpm for 10 minutes to prepare Solution A.

### (2) Preparation of Solution B

Sodium benzoate and propylene glycol were added to purified water and dissolved. The solution was then warmed to 70°C, and xanthan gum was added thereto and dissolved. The solution was then cooled to room temperature to prepare Solution B.

### (3) Preparation of Examples 1-3

Solution A and Solution B were mixed in a mass ratio of 1:1 to prepare the preparations of Examples 1-3.

**[Table 1]**

| Formulation amount (Content) | Example 1 | |
|---|---|---|
| | Solution A | Solution B |
| Compound 1 hydrochloride | 8.0 g (80 mg/mL) | - |
| hypromellose | 2.0 g (20 mg/mL) | - |
| propylene glycol | - | 10.0 g (100 mg/mL) |
| sodium benzoate | 0.2 g (2.0 mg/mL) | 0.2 g (2.0 mg/mL) |
| xanthan gum | - | 1.6 g (16 mg/mL) |
| sodium chloride | 2.0 g (20 mg/mL) | - |
| sodium alginate | 0.04 g (0.4 mg/mL) | - |
| sucralose | 0.2 g (2.0 mg/mL) | - |
| stevia | 0.2 g (2.0 mg/mL) | - |
| apple flavor | 0.04 g (0.4 mg/mL) | - |
| erythritol | 10.0 g (100 mg/mL) | - |
| purified water | 100 mL in total | 100 mL in total |

**[Table 2]**

| Formulation amount (Content) | Example 2 | |
|---|---|---|
| | Solution A | Solution B |
| Compound 1 hydrochloride | 8.0 g (80 mg/mL) | - |
| hypromellose | 2.0 g (20 mg/mL) | - |
| propylene glycol | - | 10.0 g (100 mg/mL) |
| sodium benzoate | 0.2 g (2.0 mg/mL) | 0.2 g (2.0 mg/mL) |
| xanthan gum | - | 1.0 g (10 mg/mL) |
| sodium chloride | 2.0 g (20 mg/mL) | - |
| sodium alginate | 0.2 g (2.0 mg/mL) | - |
| sucralose | 0.2 g (2.0 mg/mL) | - |
| stevia | 0.2 g (2.0 mg/mL) | - |
| apple flavor | 0.04 g (0.4 mg/mL) | - |
| erythritol | 10.0 g (100 mg/mL) | - |
| purified water | 100 mL in total | 100 mL in total |

**[Table 3]**

| Formulation amount (Content) | Example 3 | |
|---|---|---|
| | Solution A | Solution B |
| Compound 1 hydrochloride | 8.0 g (80 mg/mL) | - |
| hypromellose | 2.0 g (20 mg/mL) | - |
| propylene glycol | - | 10.0 g (100 mg/mL) |
| sodium benzoate | 0.2 g (2.0 mg/mL) | 0.2 g (2.0 mg/mL) |
| xanthan gum | - | 1.6 g (16 mg/mL) |
| sodium chloride | 2.0 g (20 mg/mL) | - |
| sodium alginate | 0.08 g (0.8 mg/mL) | - |
| sucralose | 0.2 g (2.0 mg/mL) | - |
| stevia | 0.2 g (2.0 mg/mL) | - |
| apple flavor | 0.04 g (0.4 mg/mL) | - |
| erythritol | 10.0 g (100 mg/mL) | - |
| purified water | 100 mL in total | 100 mL in total |

The formulation amount and content of each ingredient in the preparations of the above Examples are shown in Table below.

**[Table 4]**

| Formulation amount (Content) | Example 1 | Example 2 |
|---|---|---|
| Compound 1 hydrochloride | 8.0 g (40 mg/mL) | 8.0 g (40 mg/mL) |
| hypromellose | 2.0 g (10 mg/mL) | 2.0 g (10 mg/mL) |
| propylene glycol | 10.0 g (50 mg/mL) | 10.0 g (50 mg/mL) |
| sodium benzoate | 0.4 g (2.0 mg/mL) | 0.4 g (2.0 mg/mL) |
| xanthan gum | 1.6 g (8.0 mg/mL) | 1.0 g (5.0 mg/mL) |
| sodium chloride | 2.0 g (10 mg/mL) | 2.0 g (10 mg/mL) |
| sodium alginate | 0.04 g (0.2 mg/mL) | 0.2 g (1.0 mg/mL) |
| sucralose | 0.2 g (1.0 mg/mL) | 0.2 g (1.0 mg/mL) |
| stevia | 0.2 g (1.0 mg/mL) | 0.2 g (1.0 mg/mL) |
| apple flavor | 0.04 g (0.2 mg/mL) | 0.04 g (0.2 mg/mL) |
| erythritol | 10.0 g (50 mg/mL) | 10.0 g (50 mg/mL) |
| purified water | 200 mL in total | 200 mL in total |

**[Table 5]**

| Formulation amount (Content) | Example 3 |
|---|---|
| Compound 1 hydrochloride | 8.0 g (40 mg/mL) |
| hypromellose | 2.0 g (10 mg/mL) |
| propylene glycol | 10.0 g (50 mg/mL) |
| sodium benzoate | 0.4 g (2.0 mg/mL) |
| xanthan gum | 1.6 g (8.0 mg/mL) |
| sodium chloride | 2.0 g (10 mg/mL) |
| sodium alginate | 0.08 g (0.4 mg/mL) |
| sucralose | 0.2 g (1.0 mg/mL) |
| stevia | 0.2 g (1.0 mg/mL) |
| apple flavor | 0.04 g (0.2 mg/mL) |
| erythritol | 10.0 g (50 mg/mL) |
| purified water | 200 mL in total |

### Examples 4-6

According to the following procedure, the preparations of Examples 4-6 were prepared from Compound 1 hydrochloride, additives and a solvent in the amounts shown in Table below.

### (1) Preparation of Solution A

Hypromellose, sodium benzoate, sodium chloride and sucralose were added to purified water and dissolved. Stevia and apple flavor were then added thereto and dissolved. In Example 4, erythritol was further added thereto and dissolved. To each solution was then added Compound 1 hydrochloride, and the mixture was dispersed with Precision Dispersion/Emulsification machine: CLEARMIX CLM-0.8S (M Technique Co., Ltd.) at 8000 rpm for 10 minutes to prepare Solution A.

### (2) Preparation of Solution B

Sodium benzoate and propylene glycol were added to purified water and dissolved. The solution was then warmed to 70°C, and xanthan gum was added thereto and dissolved. The solution was then cooled to room temperature to prepare Solution B.

### (3) Preparation of Examples 4-6

Solution A and Solution B were mixed in a mass ratio of 1:1 to prepare the preparations of Examples 4-6.

**[Table 6]**

| Formulation amount (Content) | Example 4 | |
|---|---|---|
| | Solution A | Solution B |
| Compound 1 hydrochloride | 8.0 g (80 mg/mL) | - |
| hypromellose | 2.0 g (20 mg/mL) | - |
| propylene glycol | - | 10.0 g (100 mg/mL) |
| sodium benzoate | 0.2 g (2.0 mg/mL) | 0.2 g (2.0 mg/mL) |
| xanthan gum | - | 1.0 g (10 mg/mL) |
| sodium chloride | 2.0 g (20 mg/mL) | - |
| sucralose | 0.2 g (2.0 mg/mL) | - |
| stevia | 0.2 g (2.0 mg/mL) | - |
| apple flavor | 0.04 g (0.4 mg/mL) | - |
| erythritol | 30.0 g (300 mg/mL) | - |
| purified water | 100 mL in total | 100 mL in total |

**[Table 7]**

| Formulation amount (Content) | Example 5 | |
|---|---|---|
| | Solution A | Solution B |
| Compound 1 hydrochloride | 8.0 g (80 mg/mL) | - |
| hypromellose | 2.0 g (20 mg/mL) | - |
| propylene glycol | - | 10.0 g (100 mg/mL) |
| sodium benzoate | 0.2 g (2.0 mg/mL) | 0.2 g (2.0 mg/mL) |
| xanthan gum | - | 1.7 g (17 mg/mL) |
| sodium chloride | 2.0 g (20 mg/mL) | - |
| sucralose | 0.2 g (2.0 mg/mL) | - |
| stevia | 0.2 g (2.0 mg/mL) | - |
| apple flavor | 0.04 g (0.4 mg/mL) | - |
| purified water | 100 mL in total | 100 mL in total |

**[Table 8]**

| Formulation amount (Content) | Example 6 | |
|---|---|---|
| | Solution A | Solution B |
| Compound 1 hydrochloride | 8.0 g (80 mg/mL) | - |
| hypromellose | 2.0 g (20 mg/mL) | - |
| propylene glycol | - | 10.0 g (100 mg/mL) |
| sodium benzoate | 0.2 g (2.0 mg/mL) | 0.2 g (2.0 mg/mL) |
| xanthan gum | - | 1.8 g (18 mg/mL) |
| sodium chloride | 2.0 g (20 mg/mL) | - |
| sucralose | 0.2 g (2.0 mg/mL) | - |
| stevia | 0.2 g (2.0 mg/mL) | - |
| apple flavor | 0.04 g (0.4 mg/mL) | - |
| purified water | 100 mL in total | 100 mL in total |

The formulation amount and content of each ingredient in the preparations of the above Examples are shown in Table below.

**[Table 9]**

| Formulation amount (Content) | Example 4 Example 5 | |
|---|---|---|
| Compound 1 hydrochloride | 8.0 g (40 mg/mL) | 8.0 g (40 mg/mL) |
| hypromellose | 2.0 g (10 mg/mL) | 2.0 g (10 mg/mL) |
| propylene glycol | 10.0 g (50 mg/mL) | 10.0 g (50 mg/mL) |
| sodium benzoate | 0.4 g (2.0 mg/mL) | 0.4 g (2.0 mg/mL) |
| xanthan gum | 1.0 g (5 mg/mL) | 1.7 g (8.5 mg/mL) |
| sodium chloride | 2.0 g (10 mg/mL) | 2.0 g (10 mg/mL) |
| sucralose | 0.2 g (1.0 mg/mL) | 0.2 g (1.0 mg/mL) |
| stevia | 0.2 g (1.0 mg/mL) | 0.2 g (1.0 mg/mL) |
| apple flavor | 0.04 g (0.2 mg/mL) | 0.04 g (0.2 mg/mL) |
| erythritol | 30.0 g (150 mg/mL) | - |
| purified water | 200 mL in total | 200 mL in total |

**[Table 10]**

| Formulation amount (Content) | Example 6 |
|---|---|
| Compound 1 hydrochloride | 8.0 g (40 mg/mL) |
| hypromellose | 2.0 g (10 mg/mL) |
| propylene glycol | 10.0 g (50 mg/mL) |
| sodium benzoate | 0.4 g (2.0 mg/mL) |
| xanthan gum | 1.8 g (9.0 mg/mL) |
| sodium chloride | 2.0 g (10 mg/mL) |
| sucralose | 0.2 g (1.0 mg/mL) |
| stevia | 0.2 g (1.0 mg/mL) |
| apple flavor | 0.04 g (0.2 mg/mL) |
| purified water | 200 mL in total |

### Examples 7-8

According to the following procedure, the preparations of Examples 7-8 were prepared from Compound 1 hydrochloride, additives and a solvent in the amounts shown in Table below.

### (1) Preparation of Solution A

Hypromellose, methylparaben and dipotassium phosphate were added to purified water and dissolved. In Example 8, sucralose was further added thereto and dissolved. To each solution was then added Compound 1 hydrochloride, and the mixture was dispersed with Precision Dispersion/Emulsification machine: CLEARMIX CLM-0.3S (M Technique Co., Ltd.) at 8000 rpm for 10 minutes to prepare Solution A.

### (2) Preparation of Solution B

Methylparaben and propylene glycol were added to purified water and dissolved. The solution was then warmed to 70°C, and xanthan gum and sodium alginate were added thereto and dissolved. The solution was then cooled to room temperature to prepare Solution B.

### (3) Preparation of Examples 7-8

Solution A and Solution B were mixed in a mass ratio of 1:1 to prepare the preparations of Examples 7-8.

**[Table 11]**

| Formulation amount (Content) | Example 7 | |
|---|---|---|
| | Solution A | Solution B |
| Compound 1 hydrochloride | 8.0 g (80 mg/mL) | - |
| hypromellose | 2.0 g (20 mg/mL) | - |
| propylene glycol | - | 10.0 g (100 mg/mL) |
| methylparaben | 0.25 g (2.5 mg/mL) | 0.25 g (2.5 mg/mL) |
| xanthan gum | - | 0.8 g (8.0 mg/mL) |
| dipotassium phosphate | 3.12 g (31.2 mg/mL) | - |
| sodium alginate | - | 1.2 g (12 mg/mL) |
| purified water | 100 mL in total | 100 mL in total |

**[Table 12]**

| Formulation amount (Content) | Example 8 | |
|---|---|---|
| | Solution A | Solution B |
| Compound 1 hydrochloride | 8.0 g (80 mg/mL) | - |
| hypromellose | 2.0 g (20 mg/mL) | - |
| ropylene glycol | - | 10.0 g (100 mg/mL) |
| methylparaben | 0.2 g (2.0 mg/mL) | 0.2 g (2.0 mg/mL) |
| xanthan gum | - | 0.8 g (8.0 mg/mL) |
| dipotassium phosphate | 3.12 g (31.2 mg/mL) | - |
| sodium alginate | - | 0.6 g (6.0 mg/mL) |
| sucralose | 0.012 g (0.12 mg/mL) | - |
| purified water | 100 mL in total | 100 mL in total |

The formulation amount and content of each ingredient in the preparations of the above Examples are shown in Table below.

**[Table 13]**

| Formulation amount (Content) | Example 7 | Example 8 |
|---|---|---|
| Compound 1 hydrochloride | 8.0 g (40 mg/mL) | 8.0 g (40 mg/mL) |
| hypromellose | 2.0 g (10 mg/mL) | 2.0 g (10 mg/mL) |
| propylene glycol | 10.0 g (50 mg/mL) | 10.0 g (50 mg/mL) |
| methylparaben | 0.5 g (2.5 mg/mL) | 0.4 g (2.0 mg/mL) |
| xanthan gum | 0.8 g (4.0 mg/mL) | 0.8 g (4.0 mg/mL) |
| dipotassium phosphate | 3.12 g (15.6 mg/mL) | 3.12 g (15.6 mg/mL) |
| sodium alginate | 1.2 g (6.0 mg/mL) | 0.6 g (3.0 mg/mL) |
| sucralose | - | 0.012 g (0.06 mg/mL) |
| purified water | 200 mL in total | 200 mL in total |

### Examples 9-10

According to the following procedure, the preparations of Examples 9-10 were prepared from Compound 1 hydrochloride, additives and a solvent in the amounts shown in Table below.

### (1) Preparation of Solution A

Hypromellose and sodium benzoate were added to purified water and dissolved. To the solution was then added Compound 1 hydrochloride, and the mixture was dispersed with Precision Dispersion/Emulsification machine: CLEARMIX CLM-0.8S (M Technique Co., Ltd.) at 8000 rpm for 10 minutes to prepare Solution A.

### (2) Preparation of Solution B

Sodium benzoate was added to purified water and dissolved. The solution was then warmed to 70°C, and xanthan gum and carrageenan or gellan gum were added thereto and dissolved. The solution was then cooled to room temperature to prepare Solution B.

### (3) Preparation of Examples 9-10

Solution A and Solution B were mixed in a mass ratio of 1:1 to prepare the preparations of Examples 9-10.

**[Table 14]**

| Formulation amount (Content) | Example 9 | |
|---|---|---|
| | Solution A | Solution B |
| Compound 1 hydrochloride | 8.0 g (80 mg/mL) | - |
| hypromellose | 2.0 g (20 mg/mL) | - |
| sodium benzoate | 0.2 g (2.0 mg/mL) | 0.2 g (2.0 mg/mL) |
| xanthan gum | - | 1.0 g (10 mg/mL) |
| carrageenan | - | 0.6 g (6.0 mg/mL) |
| purified water | 100 mL in total | 100 mL in total |

**[Table 15]**

| Formulation amount (Content) | Example 10 | |
|---|---|---|
| | Solution A | Solution B |
| Compound 1 hydrochloride | 8.0 g (80 mg/mL) | - |
| hypromellose | 2.0 g (20 mg/mL) | - |
| sodium benzoate | 0.2 g (2.0 mg/mL) | 0.2 g (2.0 mg/mL) |
| xanthan gum | - | 1.0 g (10 mg/mL) |
| gellan gum | - | 0.6 g (6.0 mg/mL) |
| purified water | 100 mL in total | 100 mL in total |

The formulation amount and content of each ingredient in the preparations of the above Examples are shown in Table below.

**[Table 16]**

| Formulation amount (Content) | Example 9 | Example 10 |
|---|---|---|
| Compound 1 hydrochloride | 8.0 g (40 mg/mL) | 8.0 g (40 mg/mL) |
| hypromellose | 2.0 g (10 mg/mL) | 2.0 g (10 mg/mL) |
| sodium benzoate | 0.4 g (2.0 mg/mL) | 0.4 g (2.0 mg/mL) |
| xanthan gum | 1.0 g (5.0 mg/mL) | 1.0 g (5.0 mg/mL) |
| carrageenan | 0.6 g (3.0 mg/mL) | - |
| gellan gum | - | 0.6 g (3.0 mg/mL) |
| purified water | 200 mL in total | 200 mL in total |

### Comparative Example 1

According to the following procedure, the preparation of Comparative Example 1 was prepared from Compound 1 hydrochloride, additives and a solvent in the amounts shown in Table below.

### (1) Preparation of Solution A

Sodium benzoate was added to purified water at about 70°C and dissolved, and then the solution was cooled to room temperature. Hypromellose was then added thereto and dissolved. To the solution was then added Compound 1 hydrochloride, and the mixture was dispersed with Precision Dispersion/Emulsification machine: CLEARMIX CLM-0.8S (M Technique Co., Ltd.) at 8000 rpm for 10 minutes to prepare Solution A.

### (2) Preparation of Solution B

Sodium benzoate was added to purified water at about 70°C and dissolved. The solution was then cooled to room temperature to prepare Solution B.

### (3) Preparation of Comparative Example 1

Solution A and Solution B were mixed in a mass ratio of 1:1 to prepare the preparation of Comparative Example 1.

**[Table 17]**

| Formulation amount (Content) | Comparative Example 1 | |
|---|---|---|
| | Solution A | Solution B |
| Compound 1 | 8.0 g (80 mg/mL) | - |
| hydrochloride | | |
| hypromellose | 2.0 g (20 mg/mL) | - |
| sodium benzoate | 0.2 g (2.0 mg/mL) | 0.2 g (2.0 mg/mL) |
| purified water | 100 mL in total | 100 mL in total |

The formulation amount and content of each ingredient in the preparation of the above Comparative Example are shown in Table below.

**[Table 18]**

| Formulation amount (Content) | Comparative Example 1 |
|---|---|
| Compound 1 hydrochloride | 8.0 g (40 mg/mL) |
| hypromellose | 2.0 g (10 mg/mL) |
| sodium benzoate | 0.4 g (2.0 mg/mL) |
| purified water | 200 mL in total |

### Test Example 1 (Dissolution rate of Compound 1 or salt thereof in test solution simulating the fasted stomach)

According to the dissolution test (the paddle method) described in the Japanese Pharmacopoeia 17th Edition, the aqueous suspension pharmaceutical preparations were tested under the following [Test Condition]. The amount of Compound 1 or salt thereof dissolved in each collected solution was quantified by liquid chromatography.

### [Test Condition]

Test solution: Mixture of the dissolution test solution 1 described in the Japanese Pharmacopoeia 17th Edition (50 mL) and water (200 mL)
Volume of test solution: 250 mL
Temperature of test solution: 36.5 - 37.5°C
Rotation speed: 50 rpm
Test preparation: Preparation comprising 40 mg of Compound 1, a pharmaceutically acceptable acid addition salt thereof, or a mixture thereof in terms of Compound 1 hydrochloride Sampling time of test solution: 5 minutes, 10 minutes, 15 minutes and 30 minutes after the start of test

**[Table 19]**

| | Time after the start of test (minute) | | | |
|---|---|---|---|---|
| | Dissolution ratio of Compound 1 or salt thereof in the test solution simulating the fasted stomach (%) | | | |
| | 5 | 10 | 15 | 30 |
| Example 1 | 21.1 | 29.6 | 46.1 | 84.1 |
| Example 2 | 57.8 | 73.4 | 84.6 | 97.4 |
| Example 3 | 22.9 | 33.4 | 46.0 | 81.3 |
| Example 4 | 63.5 | 91.5 | 100.9 | 104.6 |
| Example 5 | 33.1 | 42.1 | 48.2 | 66.2 |
| Example 7 | 25.8 | 33.1 | 39.1 | 50.1 |
| Example 8 | 5 6.8 | 60.1 | 69.8 | 87.0 |
| Example 9 | 48.8 | 60.8 | 66.8 | 75.6 |
| Example 10 | 19.7 | 28.3 | 34.9 | 47.0 |
| Comparative Example 1 | 100.0 | 101.0 | 101.3 | 101.8 |

### INDUSTRIAL APPLICABILITY

The present invention shows the similar absorption process to tablet and capsule in the simulated stomach when the aqueous suspension pharmaceutical preparation comprising Compound 1 or salt thereof as an active ingredient is administered orally, and thus can inhibit rapid dissolution and absorption of the drug. Also, the present invention can reduce the risk of unexpected side effects caused by the temporary increase in blood level of the drug.

## Claims

1. An aqueous suspension pharmaceutical preparation comprising the following (1) to (4):
(1) (3aR,4S,7R,7aS)-2-{(1R,2R)-2-[4-(1,2-benzisothiazol-3-yl)piperazin-1-ylmethyl]cyclohexylmethyl}hexahydro-4,7-methano-2H-isoindole-1,3-dione (Compound 1), a pharmaceutically acceptable acid addition salt thereof, or a mixture thereof,
(2) xanthan gum,
(3) a dispersant, and
(4) water.

2. The pharmaceutical preparation according to claim 1, which comprises an acidic polymer or salt thereof, a sugar alcohol or a mixture thereof besides the (1) to (4) defined in claim 1.

3. The pharmaceutical preparation according to claim 1 or 2, which comprises at least one ingredient selected from the group consisting of a cellulose derivative, a sucrose fatty acid ester, and polyvinyl alcohol as the dispersant.

4. The pharmaceutical preparation according to claim 3, which comprises a cellulose derivative as the dispersant.

5. The pharmaceutical preparation according to claim 1 or 2, which comprises at least one ingredient selected from the group consisting of hypromellose, hydroxypropylcellulose, and hydroxyethyl cellulose as the dispersant.

6. The pharmaceutical preparation according to claim 5, which comprises hypromellose as the dispersant.

7. The pharmaceutical preparation according to any one of claims 2 to 6, which comprises at least one ingredient selected from the group consisting of (i) at least one acidic polymer or salt whereof selected from the group consisting of alginic acid, carrageenan, gelatin, guar gum, and pectin, and (ii) at least one sugar alcohol selected from the group consisting of erythritol and sorbitol.

8. The pharmaceutical preparation according to claim 7, which comprises at least one ingredient selected from the group consisting of sodium alginate and erythritol.

9. The pharmaceutical preparation according to any one of claims 1 to 8 with a pH of 2.5 - 5.5.

10. The pharmaceutical preparation according to any one of claims 1 to 9, which further comprises (5) one or more chlorides selected from an inorganic chloride or a quaternary ammonium chloride having 4 to 12 carbon atoms.

11. The pharmaceutical preparation according to any one of claims 1 to 10, wherein the concentration of xanthan gum is about 3 mg/mL - about 15 mg/mL.

12. The pharmaceutical preparation according to any one of claims 1 to 10, wherein the concentration of xanthan gum is about 4 mg/mL - 9 mg/mL.

13. The pharmaceutical preparation according to any one of claims 1 to 12, wherein the content of Compound 1, a pharmaceutically acceptable acid addition salt thereof, or a mixture thereof is about 5 - about 120 mg/mL in terms of Compound 1 hydrochloride.

14. The pharmaceutical preparation according to any one of claims 1 to 12, wherein the content of Compound 1, a pharmaceutically acceptable acid addition salt thereof, or a mixture thereof is about 10 - about 100 mg/mL in terms of Compound 1 hydrochloride.

15. The pharmaceutical preparation according to any one of claims 1 to 12, wherein the content of Compound 1, a pharmaceutically acceptable acid addition salt thereof, or a mixture thereof is about 20 - about 80 mg/mL in terms of Compound 1 hydrochloride.

16. The pharmaceutical preparation according to any one of claims 1 to 15, wherein Compound 1, a pharmaceutically acceptable acid addition salt thereof, or a mixture thereof is Compound 1 hydrochloride.

17. The pharmaceutical preparation according to any one of claims 1 to 16 for oral administration.

18. The pharmaceutical preparation according to any one of claims 1 to 17, wherein the dissolution ratio of Compound 1, a pharmaceutically acceptable acid addition salt thereof, or a mixture thereof at 5 minutes after the start of the test performed under the following test condition according to the dissolution test (the paddle method) described in the Japanese Pharmacopoeia 17th Edition is 70% or less.
[Test Condition]
Test solution: Mixture of the dissolution test solution 1 described in the Japanese Pharmacopoeia 17th Edition (50 mL) and water (200 mL)
Volume of test solution: 250 mL
Temperature of test solution: 36.5 - 37.5°C Rotation speed: 50 rpm
Test preparation: Preparation comprising 40 mg of Compound 1, a pharmaceutically acceptable acid addition salt thereof, or a mixture thereof in terms of Compound 1 hydrochloride

19. The pharmaceutical preparation according to any one of claims 1 to 17, wherein the dissolution ratio of Compound 1, a pharmaceutically acceptable acid addition salt thereof, or a mixture thereof at 5 minutes after the start of the dissolution test according to claim 18 is 60% or less.

20. The pharmaceutical preparation according to any one of claims 1 to 17, wherein the dissolution ratio of Compound 1, a pharmaceutically acceptable acid addition salt thereof, or a mixture thereof at 5 minutes after the start of the dissolution test according to claim 18 is 50% or less.

21. A medicament for treatment and/or prophylaxis of a psychiatric disease, which comprises the pharmaceutical preparation according to any one of claims 1 to 20.

22. The medicament according to claim 21, wherein the psychiatric disease is schizophrenia, bipolar disorder, senile dementia, or depression.

23. A method of treating and/or preventing a psychiatric disease, which comprises administering the pharmaceutical preparation according to any one of claims 1 to 20 to a patient in need thereof.

24. The method according to claim 23, wherein the psychiatric disease is schizophrenia, bipolar disorder, senile dementia, or depression.

25. Use of the pharmaceutical preparation according to any one of claims 1 to 20 in the manufacture of a medicament for treatment and/or prophylaxis of a psychiatric disease.

26. The use according to claim 25, wherein the psychiatric disease is schizophrenia, bipolar disorder, senile dementia, or depression.

27. The pharmaceutical preparation according to any one of claims 1 to 20 for use in the treatment and/or prophylaxis of a psychiatric disease.

28. The pharmaceutical preparation according to claim 27, wherein the psychiatric disease is schizophrenia, bipolar disorder, senile dementia, or depression.

29. An agent for controlling dissolution of an aqueous suspension pharmaceutical preparation, comprising xanthan gum.

30. The agent according to claim 29, wherein the dissolution control is the inhibition of the initial dissolution rate.

31. The agent according to claim 29, wherein the dissolution control is the inhibition of the initial dissolution rate in the solution at a pH of 1.0 - 2.5.

32. The agent according to any one of claims 29 to 31, which further comprises one ingredient selected from an acidic polymer or salt thereof or a sugar alcohol, or a mixture thereof.

33. An agent for controlling dissolution of an aqueous suspension pharmaceutical preparation with a pH of 2.5 - 5.5 comprising the following (1) to (4):
(1) (3aR,4S,7R,7aS)-2-{(1R,2R)-2-[4-(1,2-benzisothiazol-3-yl)piperazin-1-ylmethyl]cyclohexylmethyl}hexahydro-4,7-methano-2H-isoindole-1,3-dione (Compound 1), a pharmaceutically acceptable acid addition salt thereof, or a mixture thereof,
(2) one or more chlorides selected from an inorganic chloride or a quaternary ammonium chloride having 4 to 12 carbon atoms,
(3) a dispersant, and
(4) water,
wherein the agent for controlling dissolution comprises xanthan gum.

34. The agent according to claim 33, which further comprises an acidic polymer or salt thereof, a sugar alcohol, or a mixture thereof.

35. A method of controlling the dissolution of an aqueous suspension pharmaceutical preparation, which comprises adding xanthan gum to the aqueous suspension pharmaceutical preparation.

36. The method according to claim 35, wherein the dissolution control is the inhibition of the initial dissolution rate.
